# FASCICULE DE BREVET EUROPEEN

(11) **EP 1 961 489 B1**
(45) Date de publication et mention de la délivrance du brevet: **11.02.2015**
(21) Numéro de dépôt: 08151852.4
(22) Date de dépôt: 22.02.2008
(51) Int. Cl.: B05B 17/06

(54) **Dispositif de pulvérisation d'une composition de brillance**
Vorrichtung zum Versprühen einer Glanzzusammensetzung
Device for spraying a gloss composition

(30) Priorité: 23.02.2007 FR 0753480
(43) Date de publication de la demande: 27.08.2008
(73) Titulaire: L'Oréal, 75008 Paris (FR)
(72) Inventeur: Le Bourhis, Francis, 93300 Aubervilliers (FR)
(74) Mandataire: Caillet, Isabelle

(56) Documents cités:
- EP-A- 1 561 484
- WO-A-95/15822
- US-A1- 2006 032 941

## Description

La présente invention se rapporte à un dispositif de pulvérisation spécifique, sous forme de spray, comprenant une composition cosmétique comprenant elle-même au moins une silicone, et/ou au moins un corps gras, destinée à apporter de la brillance à la chevelure.

Les produits de brillance en aérosol commercialisés à ce jour sont généralement composés d'une phase liquide contenant au moins une matière première apportant de la brillance, telle qu'une silicone ou un corps gras et propulsée par un gaz liquéfié sous pression réduite, générateur d'aérosol. De tels gaz comprennent généralement des composés organiques volatiles (COV).

La mise en place de nouvelles législations tend à imposer une diminution de la quantité de composés organiques volatiles (COV) relâchés dans l'atmosphère par les gaz générateurs d'aérosols.

Pour parvenir à cette diminution, un gaz propulseur non COV tel que le HFA 152a peut être utilisé pour substituer partiellement ou totalement les gaz propulseurs utilisés habituellement. Néanmoins, l'utilisation de ce type de gaz n'est pas autorisée dans tous les pays.

Dans le cas des aérosols classiques, une autre solution pour limiter l'usage des COV, consiste à introduire une importante quantité d'eau dans les compositions cosmétiques.

Les résultats en terme de qualité d'usage et d'efficacité cosmétique de compositions cosmétiques comprenant une importante quantité d'eau, sont en retrait par rapport aux formulations antérieures. En effet, la présence d'eau, peu compatible avec la présence des agents apportant de la brillance, altère le niveau de brillance.

Un autre moyen de limiter l'usage des COV est de ne pas employer de gaz de propulsion, en ayant recours à un dispositif de pulvérisation équipé d'une pompe mécanique. A chaque actionnement du dispositif, une dose unitaire de produit est délivrée sur la chevelure. Cette discontinuité du débit peut entraîner une répartition inégale du produit sur la chevelure et un niveau de brillance médiocre de la coiffure.

De manière surprenante, la demanderesse a découvert que l'utilisation d'un dispositif de pulvérisation spécifique, comprenant un récipient dont la buse comporte une membrane perforée, actionnée par un système produisant des vibrations et au travers de laquelle la composition cosmétique liquide se transforme en gouttelettes, le système produisant des vibrations étant notamment un système piézoélectrique, permet de résoudre les problèmes mentionnés ci-dessus lorsqu'il est utilisé conjointement avec une composition cosmétique contenant au moins une silicone, et/ou au moins un corps gras non siliconé. Cette utilisation conjointe conduit, sans avoir recours à des gaz propulseurs générateurs de COV, à un excellent niveau de brillance, uniforme, par rapport aux solutions de l'art antérieur décrites ci-dessus.

L'invention a par conséquent pour objet un dispositif de pulvérisation d'une composition cosmétique comprenant un récipient équipé d'un mécanisme de pulvérisation comprenant :
- une membrane perforée, les perforations de la membrane faisant communiquer l'intérieur du récipient avec l'environnement externe,
- un actionneur pour faire vibrer la membrane,
- un moyen pour amener la composition cosmétique liquide contenue dans le récipient au contact d'une surface interne de la membrane, tel que cela est divulgué dans le document WO95/15822, formant ainsi le préambule de la revendication 1, au-delà des termes du préambule la composition cosmétique, sous l'effet des vibrations de la membrane s'écoulant à travers les perforations jusqu'à une surface externe de la membrane d'où elle émerge sous forme de gouttelettes, et
la composition cosmétique liquide comprenant, dans un milieu cosmétiquement acceptable 0,1 à 40% en poids d'au moins une silicone, et/ou 0,1 à 40% en poids d'au moins un corps gras non siliconé, par rapport au poids total de la composition cosmétique.

Les perforations dans la membrane ont de préférence, une conicité inversée, c'est-à-dire une surface en coupe transversale plus grande sur la surface externe de la membrane, faisant face à l'environnement externe, que sur la surface interne, faisant face à l'intérieur du récipient.

Le dispositif de pulvérisation peut comprendre en outre un moyen de décalage de pression, tel que décrit dans la demande WO95/15822, fournissant une pression réduite au liquide en contact avec la surface interne de la membrane. La pression réduite peut varier d'une pression nulle jusqu'à la pression à laquelle l'air est aspiré à travers les perforations de la membrane en contact avec la composition.

De préférence, les perforations, sur la surface externe de la membrane, ne se touchent pas.

De préférence, l'actionneur est un actionneur piézo-électrique, par exemple conçu pour faire vibrer la membrane dans une gamme de fréquence allant de 20 KHz à 7 MHz. L'énergie nécessaire au fonctionnement de l'actionneur piézo-électrique peut être obtenue grâce à un générateur électrique, par exemple une pile électrique, une batterie ou une cellule photovoltaïque pouvant être éventuellement couplé à un circuit électronique.

Dans le mécanisme de pulvérisation défini ci-dessus, le moyen pour amener la composition cosmétique liquide à la surface de la membrane peut comprendre un mécanisme d'alimentation par capillarité ou alternativement, un mécanisme d'alimentation à générateur de bulles. De tels mécanismes sont décrits par exemple dans la demande internationale WO95/15822.

Dans des modes de réalisations particuliers de l'invention, toutes les perforations ont une conicité inversée ou à l'inverse, la membrane comporte, de plus, des perforations de conicité normale.

Par perforation de conicité normale, on entend au sens de la présente invention des perforations dont la surface en coupe transversale est plus petite sur la surface externe de la membrane, faisant face à l'environnement externe, que sur la surface interne, faisant face à l'intérieur du récipient.

Lorsque des perforations de conicité normale sont présentes, celles-ci sont de préférence disposées autour et à l'extérieur des perforations de conicité inversée.

Le moyen pour amener la composition cosmétique liquide à la surface de la membrane peut être conçu pour amener ladite composition sur la surface interne de ladite membrane, ou à l'inverse, être conçu pour amener ladite composition sur la surface externe de ladite membrane. De telles variantes du dispositif de pulvérisation sont décrites, par exemple, dans la demande internationale WO95/15822.

A titre d'exemple, la membrane peut être formée d'un disque circulaire d'un diamètre de 8 mm, de nickel électroformé, d'une épaisseur de 70µm possédant une pluralité de perforations. Les perforations peuvent présenter une surface en coupe transversale en forme de disque circulaire dont le diamètre varie de 4 à 150µm sur la surface externe de la membrane, faisant face à l'environnement externe, et sur la surface interne, faisant face à l'intérieur du récipient, une surface en coupe transversale en forme de disque circulaire dont le diamètre varie de 2 à 50µm et par exemple, de 10 à 20µm.

Lors de l'utilisation du dispositif, la composition cosmétique émerge sous forme de gouttelettes dont le diamètre moyen est compris de préférence entre 20 et 100µm, et de manière encore plus préférée entre 30 et 60 µm.

Les silicones utilisables dans les compositions cosmétiques de la présente invention, sont de préférence des silicones volatiles ou non volatiles, cycliques, linéaires ou ramifiées, modifiées ou non par des groupements organiques, ou des silicones arylées, ayant de préférence une viscosité de 5.10⁻⁶ à 2,5m²/s à 25°C et de préférence 1.10⁻⁵ à 1m²/s.

Les silicones utilisables conformément à l'invention peuvent être sous forme solubles, dispersée, micro ou nano dispersée dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi :
(i) les polydiolkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATINE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la "SILICONE VOLATILE FZ 3109" commercialisée par la société UNION CARBIDE, de formule :
avec D" :

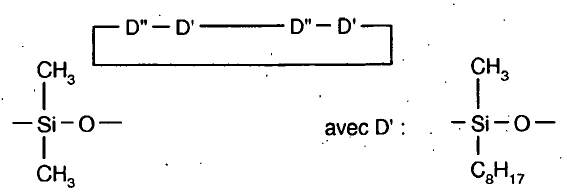

On peut également citer les mélanges de polydialkyl siloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne (ou diméthiconol (CTFA) et d'un poly-diméthylsiloxane cyclique également appelé cyclométhicone (CTFA) tel que le produit Q2 1401 commercialisé par la société DOW CORNING ;
- les mélanges d'une gomme polydiméthyl-siloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2}
dans lesquelles R représente un alkyle possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement préférées et choisies parmi les polydiméthyl méthylphénylsiloxanes, les polydiméthyl diphénylsiloxanes linéaires et/ou ramifiés de viscosité allant de 1.10⁻⁵ à 5.10⁻²m²/s à 25 °C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- une phényl triméthylsiloxy trisiloxane, en particulier l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

Dans une première variante préférée de l'invention la ou les silicones de l'invention sont choisies parmi les polydiméthylsiloxanes volatiles, linéaires ou cycliques.

Dans une seconde variante préférée la ou les silicones de l'invention sont choisies parmi les silicones phénylées.

Les silicones telles que décrites ci-dessus peuvent être utilisées seules ou en mélange, en une quantité comprise entre 0,1 et 40% en poids, de préférence entre 1 et 20% en poids par rapport au poids de total de la composition.

Par corps gras non siliconé, on entend, au sens de la présente invention, un composé choisi parmi un alcool gras, un ester gras, une huile minérale, végétale, animale ou synthétique, ou une cire.

Comme huiles non siliconées utilisables dans la composition de l'invention, on peut notamment citer, par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que Parléam^{®} ; les isoparaffines comme l'isohexadécane et l'isodécane.
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M ;

Le ou les alcools gras sont choisis notamment parmi les alcools gras saturés ou insaturés, linéaires ou ramifiés ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les esters gras sont notamment les esters d'acides carboxyliques en particulier les esters mono, di, tri ou tétracarboxyliques.

Les esters d'acides monocarboxyliques sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆ , le nombre total de carbone des esters étant supérieur ou égal à 10.

Parmi ces esters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C₄-C₂₂ et d'alcools en Cₗ-C₂₂ et les esters d'acides mono di ou tricarboxyliques et d'alcools di, tri, tétra ou pentahydroxy en C₂-C₂₆.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

De préférence, parmi les corps gras non siliconés on utilisera l'huile de vaseline ou un ester d'acide monocarboxylique.

Le ou les corps gras non siliconés représentent 0,1 à 40% en poids, de préférence 1 à 20% en poids par rapport au poids total de la composition cosmétique.

Les compositions cosmétiques, décrites ci-dessus peuvent également contenir d'autres actifs cosmétiquement acceptables, tels que par exemple des polymères fixants, des agents tensioactifs, des agents épaississants, des agents de pénétration, des parfums, des peptisants, des tampons, et divers adjuvants usuels comme des filtres UV, des conservateurs, des céramides, des pseudocéramides, les vitamines ou provitamines comme le panthénol, des agents réducteurs, des émulsionnants, des conservateurs, des charges, des filtres solaires, des protéines, des agents hydratants, des émollients, des agents adoucissants, des agents anti-mousse, des agents anti-radicaux libres, des bactéricides, des séquestrants, des anti-pelliculaires, des anti-oxydants, des agents alcalinisants, des polyols, et tout autre additif classiquement utilisé dans les compositions cosmétiques destinées à être appliquées sur les cheveux.

Par polymère fixant, on entend au sens de la présente invention tout polymère susceptible d'apporter et/ou de maintenir une forme à une chevelure.

On peut utiliser pour la présente invention tout polymère fixant connu en tant que tel dans le domaine des traitements capillaires, ainsi que bien entendu également des mélanges contenant plusieurs de ces polymères. On distingue classiquement les polymères fixants cationiques, anioniques, amphotères et non ioniques.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs suivants : 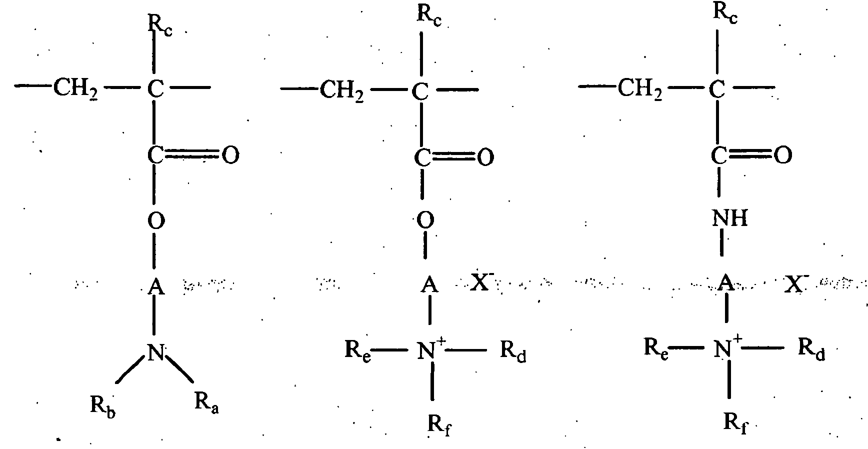 dans lesquels :
   Rₐ et R_{b} représentent chacun un atome d'hydrogène ou un groupe alkyle en C₁₋₆,
   R_{c} désigne un atome d'hydrogène ou un radical CH₃.
   R_{d}, Rₑ et R_{f}, identiques ou différents, représentent chacun un groupe alkyle en C₁₋₁₈ ou un radical benzyle,
   A est un groupe alkyle linéaire ou ramifié en C₁₋₆ ou un groupe hydroxyalkyle en C₁₋₄, et
   X⁻ désigne un anion méthosulfate ou halogénure tel qu'un ion chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieurs, des acides acryliques ou méthacryliques ou leurs esters, des vinyllactames tels que la vinylpyrrolidone ou le vinylcaprolactame, et des esters vinyliques.
   Ainsi, on peut citer parmi les copolymères de la famille (1) :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la Société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT® P 100 par la Société CIBA GEIGY,
   - le copolymère d'acrylamide et de méthosulfate de méthacryloyloxyéthyltriméthylammonium vendu sous la dénomination RETEN® par la Société HERCULES,
   - les copolymères vinylpyrrolidone/acrylate ou méthacrylate de dialkylaminoalkyle quaternisés ou non, tels que les produits vendus sous la dénomination GAFQUAT® par la Société ISP, comme par exemple GAFQUAT® 734 OU GAFQUAT® 755, ou bien les produits dénommés COPOLYMER® 845 , 958 et 937. Ces polymères sont décrits en détail dans les demandes de brevets français FR2.077.143 et FR2.393.573,
   - les terpolymères méthacrylate de diméthylaminoéthyle/vinylcaprolactame/vinylpyrrolidone tels que le produit commercialisé sous la dénomination GAFFIX VC 713 par la Société ISP, et
   - le copolymère vinylpyrrolidone/méthacrylamide de diméthylaminopropyle quaternisé tel que le produit vendu sous la dénomination GAFQUAT® HS 100 par la Société ISP.
(2) les polysaccharides quaternisés décrits plus particulièrement par les brevets américains 3.589.578 et 4.031.307 tels que les gommes de guar contenant des groupements cationiques trialkylammonium.
   De tels produits sont commercialisés notamment sous les dénominations commerciales de JAGUAR® C13 S, JAGUAR® C15 et JAGUAR® C17 par la Société MEYHALL.
(3) Les copolymères quaternaires de vinylpyrrolidone et de vinylimidazole tels que les produits commercialisés par BASF sous la dénomination LUVIQUAT® TFC,
(4) les chitosanes ou leurs sels, en particulier les acétate, lactate, glutamate, gluconate ou pyrrolidonecarboxylate de chitosane.
   On peut citer le chitosane ayant un taux de désacétylation de 90,5% en poids vendu sous la dénomination KYTAN BRUT STANDARD® par la Société ABER TECHNOLOGIES, le pyrrolidonecarboxylate de chitosane vendu sous la dénomination KYTAMER® PC par la Société AMERCHOL.
(5) Les dérivés de cellulose cationiques tels que les copolymères de cellulose ou les dérivés de cellulose greffés avec un monomère hydrosoluble comportant un groupe ammonium quaternaire et décrits notamment dans le brevet US 4 131 576 tels que les hydroxyalkylcelluloses, comme les hydroxyméthylhydroxyéthyl- ou hydroxypropylcelluloses greffées notamment avec un sel de méthacryloyloxyéthyltriméthylammonium, de méthacrylamidopropyltriméthylammonium ou de diméthyldiallylammonium.

Les produits commercialisés répondant à cette définition sont plus particulièrement les produits vendus sous les dénominations CELQUAT® L 200 et CELQUAT® H 100 par la Société NATIONAL STARCH.

Les polymères fixants anioniques généralement utilisés sont des polymères comportant des groupements dérivés d'un acide carboxylique, sulfonique ou phosphorique et ont un poids moléculaire moyen en poids compris entre environ 500 et 5 000 000.

Les groupements acides carboxyliques sont apportés par des monomères insaturés contenant une ou deux fonctions acide carboxylique, tels que ceux répondant à la formule : 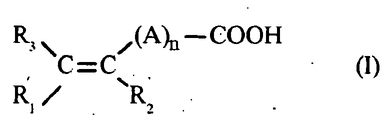 dans laquelle n est un nombre entier de 0 à 10, A désigne un groupement méthylène, éventuellement relié à l'atome de carbone du groupement insaturé ou du groupement méthylène voisin, lorsque n est supérieur à 1, par l'intermédiaire d'un hétéroatome tel que oxygène ou soufre, R₃ désigne un atome d'hydrogène, un groupement phényle ou benzyle, R₁ désigne un atome d'hydrogène, un groupement alkyle inférieur ou carboxyle, et R₂ désigne un atome d'hydrogène, un groupement alkyle inférieur, un groupement -CH₂-COOH, phényle ou benzyle.

Dans la formule précitée un radical alkyle inférieur désigne de préférence un groupement ayant de 1 à 4 atomes de carbone et en particulier un groupe méthyle ou éthyle.

Les polymères fixants anioniques carboxylés préférés selon l'invention sont :
A) les homo- ou copolymères d'acide acrylique ou méthacrylique ou leurs sels et en particulier les produits commercialisés sous les dénominations VERSICOL® E ou K par la Société ALLIED COLLOID, et sous la dénomination ULTRAHOLD® par la Société BASF ; les copolymères d'acide acrylique et d'acrylamide commercialisés sous forme de sel de sodium sous les dénominations RETEN® 421, 423 ou 425 par la Société HERCULES ; les sels de sodium des acides polyhydroxycarboxyliques.
B) Les copolymères d'acide acrylique ou d'acide méthacrylique et d'un monomère monoéthylénique tel que l'éthylène, le styrène, les esters vinyliques, les esters d'acide acrylique ou méthacrylique.
   Ces copolymères peuvent être greffés sur un polyalkylène glycol tel que le polyéthylèneglycol et sont éventuellement réticulés.
   De tels polymères sont décrits en particulier dans la demande de brevet français FR 1 222 944 et dans la demande de brevet allemand DE 2 330 956. On peut notamment citer les copolymères comportant dans leur chaîne un motif acrylamide éventuellement N-alkylé et/ou hydroxyalkylé, tels que ceux décrits dans les demandes de brevet luxembourgeois LU 75370 et LU75371 ou proposés sous la dénomination QUADRAMER® par la Société AMERICAN CYANAMID.
   On peut également citer les copolymères d'acide acrylique et de méthacrylate d'alkyle en C₁-C₄, et les terpolymères de vinylpyrrolidone, d'acide (méth)acrylique et de (méth)acrylate d'alkyle en C₁-C₂₀, par exemple de lauryle (ACRYLDONE® LM de la Société ISP), de tertiobutyle (LUVIFLEX® VBM 70 commercialisé par BASF) ou de méthyle (STEPANHOLD® EXTRA commercialisé par STEPAN), et les terpolymères acide méthacrylique/acrylate d'éthyle/acrylate de tertiobutyle tel que le produit commercialisé sous la dénomination LUVIMER® 100 P par la Société BASF.
C) Les copolymères dérivés d'acide crotonique tels que ceux comportant dans leur chaîne des motifs acétate ou propionate de vinyle et éventuellement d'autres monomères tels que les esters allylique, méthallylique ou vinylique d'un acide carboxylique saturé, linéaire ou ramifié, à longue chaîne hydrocarbonée comportant au moins 5 atomes de carbone, ces polymères pouvant éventuellement être greffés et réticulés, ou encore les esters vinylique, allylique ou méthallylique d'un acide carboxylique α- ou β -cyclique.
   De tels polymères sont décrits, entre autres, dans les demandes de brevets français FR 1 222 944, FR 1 580 545, FR2 265 782, FR 2 265 781, FR 1 564 110 et FR 2 439 798.
   On peut citer à titre d'exemples de produits commerciaux entrant dans cette classe les résines 28-29-30, 26-13-14 et 28-13-10 commercialisées par la Société NATIONAL STARCH et le MEXOMERE PW proposé par la société CHIMEX.
D) Les copolymères dérivés d'acides ou d'anhydrides carboxyliques monoinsaturés en C₄- C₈ choisis parmi :
   - les copolymères comprenant :
      (ii) un ou plusieurs acides ou anhydrides maléique, fumarique, itaconique et
      (iii) au moins un monomère choisi parmi les esters vinyliques, les éthers vinyliques, les halogénures vinyliques, les dérivés phénylvinyliques, l'acide acrylique et ses esters, les fonctions anhydride de ces copolymères étant éventuellement monoestérifiées ou monoamidifiées.
      De tels polymères sont décrits en particulier dans les demandes de brevets US 2,047,398, US 2,723,248, US 2,102,113 et GB 839,805 et notamment ceux commercialisés sous les dénominations GANTREZ AN ou ES, AVANTAGE® CP par la Société ISP ;
      - les copolymères comprenant (i) un ou plusieurs anhydrides maléique, citraconique ou itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
      Ces polymères sont par exemple décrits dans les demandes de brevets français FR 2 350 384 et FR 2 357 241 de la demanderesse.
E) Les polyacrylamides comportant des groupements carboxylate.
F) Les copolymères acryliques à blocs ramifiés. Dans cette famille on peut en particulier citer les copolymères à blocs ramifiés comprenant comme monomères principaux au moins un acrylate d'alkyle en C1-C20 et/ou au moins un N mono- ou N,N di-(alkyle en C2-C12)(meth)acrylamide et de l'acide acrylique et/ou de l'acide méthacrylique. Ces polymères fixants sont des copolymères à blocs ramifiés ayant une structure constituée de blocs hydrophobes sur lesquels sont fixées, notamment par l'intermédiaire de motifs bifonctionnels, un certain nombre de blocs plus hydrophiles. Ces copolymères présentent au moins deux températures de transition vitreuse.

Ils sont notamment décrits dans la demande de brevet WO00/40628.

Les copolymères séquencés ramifiés décrits ci-dessus sont proposés par exemple sous les dénominations EX-SDR-26® et EX-SDR-45® par la société GOODRICH, et EX-SDR-752 (FIXATE G100L de NOVEON).

Ces copolymères présentent généralement la composition suivante :
Acide acrylique 26 à 36 % en moles
Acrylate de n-butyle 27,5 à 30,5 % en moles
Acide méthacrylique 33,3 à 45,3 % en moles
Méthacrylate d'allyle 0,48 à 0,92 % en moles

Les blocs les plus hydrophobes ont un poids moléculaire de 10 000 à 100 000 et les blocs les plus hydrophobes ont un poids moléculaire de 1000 à 100 000 daltons.

Les groupements anioniques des polymères fixants anioniques de la présente invention peuvent également être des groupes acide sulfonique apportés par des motifs vinylsulfonique, styrènesulfonique, naphtalènesulfonique ou acrylamidoalkylsulfonique.

Ces polymères à groupes acide sulfonique sont notamment choisis parmi :
- les sels de poly(acide vinylsulfonique) ayant un poids moléculaire moyen en poids compris entre environ 1000 et 100 000 ainsi que les copolymères d'acide vinylsulfonique et d'un comonomère insaturé tel que l'acide acrylique, l'acide méthacrylique, les esters de ces acides, l'acrylamide, les dérivés d'acrylamide, les éthers vinyliques et la vinylpyrrolidone ;
- les sels de poly(acide styrènesulfonique). On peut citer à titre d'exemple deux sels de sodium ayant un poids moléculaire moyen en poids d'environ 500 000 et d'environ 100 000 commercialisés respectivement sous les dénominations FLEXAN® 500 et FLEXAN® 130 par la Société NATIONAL STARCH. Ces composés sont décrits dans le brevet FR 2 198 719 ;
- les sels de poly(acide acrylamidesulfonique), tels que ceux mentionnés dans le brevet US 4,128,631 et plus particulièrement le poly(acide acrylamidoéthyl-propanesulfonique) commercialisé sous la dénomination COSMEDIA POLYMER® HSP 1180 par la Société HENKEL.
- Les polyesters sulfoniques linéaires.

Par polyester sulfonique, on entend des copoylesters obtenus par polycondensation d'au moins un acide dicarboxylique ou d'un de ses esters, d'au moins un diol et d'au moins un composé difonctionnel sulfoaryldicarboxylique substitué sur le noyau aromatique par un groupe - SO3M dans lequel M représente un atome d'hydrogène ou un ion métallique tel que Na+, Li+ ou K+.

Les polyesters sulfoniques linéaires dispersibles dans l'eau présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse (Tg) de ces polyesters sulfoniques est généralement comprise dans l'intervalle allant de 10 °C à 100 °C. De préférence, la Tg du ou des polyesters utilisés est supérieure ou égale à 50°C.

La température de transition vitreuse (Tg) est mesurée par analyse enthalpique différentielle (DSC, differential scanning calorimetry) selon la norme ASTM D3418-97.

Ils sont décrits plus en détail dans les demandes de brevet US 3 734 874, US 3 779 993, US 4 119 680, US 4 300 580, US 4 973 656, US 5 660 816, US 5 662 893, et US 5 674 479.

Les polyesters sulfoniques utilisés de préférence dans l'invention comprennent au moins des motifs dérivés d'acide isophtalique, de sel d'acide sulfoaryle-dicarboxylique et de diéthylèneglycol, et plus particulièrement les polyesters sulfoniques utilisés dans l'invention sont obtenus à partir d'acide isophtalique, de sel de sodium de l'acide sulfoisophtalique, de diéthylèneglycol et de 1,4-cyclohexaneméthanol.

A titre d'exemples de polyester sulfonique, on peut notamment citer ceux connus sous le nom INCI Diglycol/CHDM/Isophtalates/SIP, et vendus sous les dénominations commerciales"Eastman AQ polymer" (AQ35S, AQ38S, AQ55S, AQ48 Ultra) par la société Eastman Chemical.
De préférence, la Tg du ou des polyesters utilisés est supérieure ou égale à 50 °C.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères dérivés d'acide crotonique tels que les terpolymères acétate de vinyle/tertio-butylbenzoate de vinyle/acide crotonique (MEXOMERE PW de la société CHIMEX) et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vynyliques, des halogénures vinyliques, des dérivés phénylvinyliques, l'acide acrylique et ses esters tels que le copolymère méthylvinyléther/anhydride maléïque monoestérifié vendu sous la dénomination GANTREZ® ES 425 par la société ISP, le copolymère d'acide méthacrylique et d'acrylate d'éthyle vendu sous la dénomination LUVIMER® MAEX par la société BASF, le terpolymère de vinylpyrrolidone/acide acrylique/méthacrylate d'alkyle de lauryle vendu par la société ISP sous la dénomination ACRYLIDONE LM et le copolymère acétate de vinyle/acide crotonique vendu sous la dénomination LUVISET® CA 66 par la société BASF et le terpolymère acétate de vinyle/acide crotonique/polyéthylèneglycol sous la dénomination ARISTOFLEX® A par la société BASF, les polyesters sulfoniques linéaires et en particulier l'AQ55S de la société EASTMAN, les copolymères acryliques à blocs ramifiés et en particulier le FIXATE G100L de la société NOVEON.

Les polymères fixants amphotères utilisables pour la présente invention sont choisis notamment parmi les polymères comportant des motifs B et C répartis statistiquement dans la chaîne polymère, où B désigne un motif dérivant d'un monomère comportant au moins un atome d'azote basique et C désigne un motif dérivant d'un monomère comportant un ou plusieurs groupements acide carboxylique ou acide sulfonique. Les polymères fixants amphotères peuvent également comporter des motifs zwittérioniques de type carboxybétaïne ou sulfobétaïne. Il peut également s'agir de polymères à chaîne principale cationique comportant des groupements amine primaire, secondaire, tertiaire ou quaternaire, parmi lesquels au moins un, porte, par l'intermédiaire d'un radical hydrocarboné, un groupement acide carboxylique ou acide sulfonique. Les polymères fixants amphotères peuvent encore avoir une chaîne anionique dérivée d'acides dicarboxyliques α,β-insaturés dont l'un des groupements carbonyle a été amené à réagir avec une polyamine comportant un ou plusieurs groupements amine primaire ou secondaire.

Les polymères fixants amphotères répondant à la définition donnée ci-dessus sont choisis en particulier parmi les polymères suivants :
(1) Les polymères résultant de la copolymérisation d'un monomère vinylique portant un groupement acide carboxylique tel que l'acide acrylique, l'acide méthacrylique, l'acide maléique, l'acide α -chloroacrylique, et d'un monomère vinylique contenant au moins une fonction basique tel que les méthacrylate et acrylate de dialkylaminoalkyle ou les dialkylaminoalkyl(méth)acrylamides. De tels composés sont décrits par exemple dans la demande de brevet américain US 3,836,537.
(2) Les polymères comportant des motifs dérivés :
   (a) d'au moins un monomère choisi parmi les acrylamides ou méthacrylamides N-alkylés,
   (b) d'au moins un comonomère contenant une ou plusieurs fonctions acide carboxylique, et
   (c) d'au moins un comonomère basique tel que des esters à substituants amine primaire, secondaire, tertiaire ou quaternaire d'acide acrylique et d'acide méthacrylique, et le produit de quaternisation du méthacrylate de diméthylaminoéthyle avec le sulfate de diméthyle ou diéthyle.
   Les acrylamides ou méthacrymlamides N-alkylés (a) préférés sont ceux portant des radicaux alkyle en C₂₋₁₂ , tels que le N-éthylacrylamide, le N-tertiobutylacrylamide, le N-tertio-octylacrylamide, le N-octylacrylamide, le N-décylacrylamide, le N-dodécylacrylamide ainsi que les méthacrylamides correspondants.
   Les comonomères à groupe acide carboxylique (b) sont choisis plus particulièrement parmi les acides acrylique, méthacrylique, crotonique, itaconique, maléique, fumarique ainsi que parmi les monoesters d'alkyle en C₁₋₄ des acides ou des anhydrides maléique ou fumarique.
   Les comonomères basiques (c) préférés sont le méthacrylate d'aminoéthyle, le méthacrylate de butylaminoéthyle, le méthacrylate de N,N'-diméthylaminoéthyle et le méthacrylate de N-tertiobutylaminoéthyle.
   On utilise en particulier les copolymères dont la dénomination CTFA (4ième Ed., 1991) est « Octylacrylamide/acrylates/butylaminoéthylméthacrylate copolymer », tels que les produits commercialisés sous la dénomination AMPHOMER® ou LOVOCRYL® 47 par la Société NATIONAL STARCH.
(3) Les polyaminoamides réticulés et alcoylés, dérivés en partie ou en totalité de polyaminoamides de formule générale :

   (II) -[C(=0)-R₄-C(=0)-Z-]-

   dans laquelle R₄ représente un radical divalent dérivé d'un acide dicarboxylique saturé, d'un acide aliphatique mono- ou dicarboxylique à double liaison éthylénique, d'un ester d'alkyle en C₁₋₆ de ces acides ou d'un radical dérivant de l'addition de l'un quelconque desdits acides sur une amine bis primaire ou bis secondaire, et Z désigne un radical d'une polyalkylène-polyamine bis-primaire, mono- ou bis-secondaire, et de préférence représente :
   a) dans les proportions de 60 à 100 % en moles, le radical :

      (III) -NH-[(CH₂)ₓ-NH]ₚ-

      où x = 1 et p = 2 ou 3, ou bien x = 3 et p = 2,
      ce radical dérivant de la diéthylènetriamine, de la triéthylènetétraamine ou de la dipropylènetriamine ;
   b) dans les proportions de 0 à 40% en moles le radical de formule (III)
      dans lequel x = 2 et p = 1, dérivé de l'éthylènediamine, ou le radical 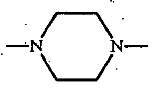 dérivé de la pipérazine :
   c) dans les proportions de 0 à 20 % en moles le radical -NH-(CH₂)₆-NH-dérivé de l'hexaméthylènediamine, ces polyaminoamines étant réticulées par addition de 0,025 à 0,35 mole par mole de groupement amine, d'un agent réticulant bifonctionnel choisi parmi les épihalohydrines, les diépoxydes, les dianhydrides, les composés di-insaturés, et alcoylés par l'acide acrylique, l'acide chloracétique ou une alcane-sulfone.
   Les acides carboxyliques saturés sont choisis de préférence parmi les acides ayant 6 à 10 atomes de carbone tels que l'acide adipique, l'acide triméthyl-2,2-4-adipique et triméthyl-2,4,4-adipique, l'acide téréphtalique, les acides à double liaison éthylénique comme par exemple les acides acrylique, méthacrylique, itaconique.
   Les alcane-sulfones utilisées dans l'alcoylation sont de préférence la propanesulfone ou la butanesulfone.
   Les sels des agents d'alcoylation sont de préférence les sels de sodium ou de potassium.
(4) Les polymères comportant des motifs zwittérionique de formule : 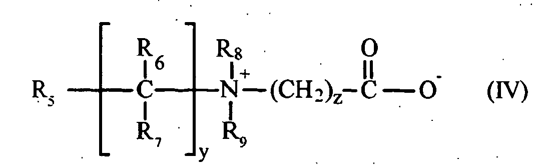 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent chacun indépendamment un atome d'hydrogène ou un groupe méthyle, éthyle ou propyle, R₈ et R₉ représentent chacun indépendamment un atome d'hydrogène ou un radical alkyle, le nombre total d'atomes de carbone dans R₈ et R₉ ne dépassant pas 10.
   Les polymères comprenant de tels motifs de formule (IV) peuvent comporter en outre des motifs dérivés de monomères non zwittérioniques tels que l'acrylate ou le méthacrylate de diméthyle ou de diéthylaminoéthyle, les acrylates ou méthacrylates d'alkyle, les acrylamides ou méthacrylamides ou l'acétate de vinyle.
   A titre d'exemple, on peut citer le copolymère méthacrylate de méthyle/méthacrylate de diméthylcarboxyméthylammonioéthyle tel que le produit commercialisé sous la dénomination DIAFORMER® Z301 par la Société SANDOZ.
(5) Les polymères dérivés du chitosane comportant des motifs monomères répondant aux formules suivantes : 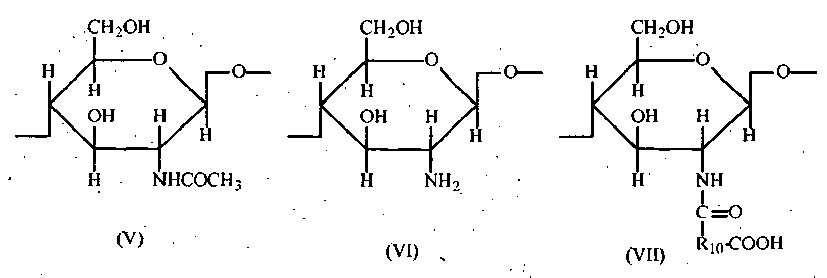 le motif de formule (V) étant présent dans des proportions comprises entre 0 et 30%, le motif de formule (VI) dans des proportions comprises entre 5 et 50% et le motif de formule (VII) dans des proportions comprises entre 30 et 90%, étant entendu que dans ce motif (VII), R₁₀ représente un radical de formule : 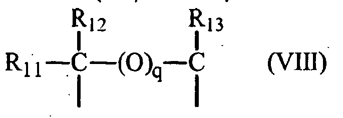 dans laquelle :
   si q = 0, alors R₁₁, R₁₂ et R₁₃, identiques ou différents, représentent chacun un atome d'hydrogène, un groupe méthyle, hydroxyle, acétoxy ou amino, un groupe monoalcoylamine ou dialcoylamine éventuellement interrompu par un ou plusieurs atomes d'azote et/ou éventuellement substitués par un ou plusieurs groupes amine, hydroxyle, carboxyle, alcoylthio ou sulfo, un groupe alcoylthio dont le groupe alcoyle porte un reste amino, l'un au moins des radicaux R₁₁, R₁₂ et R₁₃ étant dans ce cas un atome d'hydrogène ; ou si q = 1, alors R₁₁, R₁₂ et R₁₃ représentent chacun un atome d'hydrogène, ainsi que les sels formés par ces composés avec des bases ou des acides.
(6) Les polymères obtenus par N-carboxyalkylation du chitosane, comme le N-carboxyméthylchitosane ou le N-carboxybutylchitosane commercialisé sous la dénomination EVALSAN® par la Société JAN DEKKER.
(7) Les polymères répondant à la formule générale (IX) : 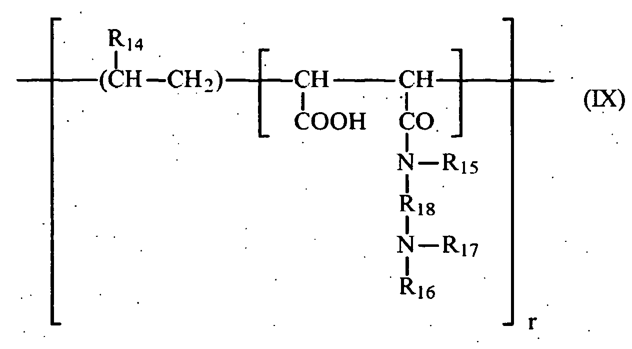 décrits notamment dans la demande de brevet FR 1 400 366, dans laquelle R₁₄ représente un atome d'hydrogène ou un radical CH₃O, CH₃CH₂O ou phényle, R₁₅ désigne un atome d'hydrogène ou un radical alkyle inférieur tel que méthyle ou éthyle, R₁₅ désigne un atome d'hydrogène ou un radical alkyle inférieur tel que méthyle ou éthyle, R₁₇ désigne un radical alkyle inférieur tel que méthyle ou éthyle ou un radical répondant à la formule :

   -R₁₈-N(R₁₆)₂,

   R₁₈ représentant un groupement -CH₂ -CH₂-,-CH₂ -CH₂- CH₂-, -CH2-CH(CH₃)-, et R₁₆ ayant les significations mentionnées ci-dessus, ainsi que les homologues supérieurs de ces radicaux contenant jusqu'à 6 atomes de carbone.
(8) Les polymères amphotères du type -D-X-D-X-choisis parmi :
   (a) les polymères obtenus par action d'acide chloroacétique ou de chloroacétate de sodium sur les composés comportant au moins un motif de formule :

      (X) -D-X-D-X-D-

      où D désigne un radical : 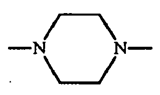 et X désigne le symbole E ou E', E ou E' identiques ou différents désignant un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote ou de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      (X') -D-X'-D-X'-

      où D désigne un radical 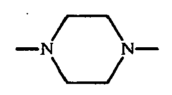
   et X' désigne le symbole E ou E' et au moins une fois E', E ayant la signification indiquée ci-dessus et E' étant un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisée par réaction avec l'acide chloroacétique ou du chloroacétate de soude.
(9) Les copolymères alkyl(C₁₋₅)vinyléther/anhydride maléique modifiés partiellement par semi-amidification avec une N,N-dialkylaminoalkylamine telle que la N,N-diméthylaminopropylamine, ou par semi-estérification avec une N,N-dialcanolamine. Ces copolymères peuvent également comporter d'autres comonomères vinyliques tels que le vinylcaprolactame.

Les polymères fixants amphotères préférés selon l'invention sont ceux de la famille (3) décrite ci-dessus, tels que ceux dont la dénomination CTFA est « Octylacrylamide/acrylates/butylaminoéthyl-méthacrylate copolymer ». On peut citer à titre d'exemple les produits commercialisés sous les dénominations AMPHOMER®, AMPHOMER® LV 71 ou LOVOCRYL® 47 par la Société NATIONAL STARCH.

D'autres polymères fixants amphotères préférés sont ceux de la famille (4), comme par exemple les copolymères de méthacrylate de méthyle et de méthacrylate de diméthylcarboxyméthylammonioéthyle, commercialisés par exemple sous la dénomination DIAFORMER® Z301 par la Société SANDOZ.

Les polymères fixants anioniques ou amphotères peuvent, si nécessaire, être partiellement ou totalement neutralisés. Les agents de neutralisation sont par exemple la soude, la potasse, l'amino-2-méthylpropanol, la monoéthanolamine, la triéthanolamine ou la triisopropanolamine, les acides minéraux ou organiques tels que l'acide chlorhydrique ou l'acide citrique.

Les polymères fixants non ioniques utilisables selon la présente invention sont choisis par exemple parmi :
- les homopolymères de vinylpyrrolidone,
- les copolymères de vinylpyrrolidone et d'acétate de vinyle,
- les polyalkyloxazolines telles que les polyéthyloxazolines proposées par la Société DOW CHEMICAL sous les dénominations PEOX® 50 000 , PEOX® 200 000 et PEOX® 500 000,
- les homopolymères d'acétate de vinyle tels que le produit proposé sous le nom de APPRETAN® EM par la Société HOECHST ou le produit proposé sous le nom de RHODOPAS® A 012 par la Société RHONE POULENC ;
- les copolymères d'acétate de vinyle et d'esters acryliques tels que le produit proposé sous la dénomination RHODOPAS® AD 310 par RHONE POULENC,
- les copolymères d'acétate de vinyle et d'éthylène tels que le produit proposé sous le nom de APPRETAN® TV par la Société HOECHST,
- les copolymères d'acétate de vinyle et d'ester maléique par exemple de maléate de dibutyle tels que le produit proposé sous le nom de APPRETAN® MB EXTRA par la Société HOECHST,
- les copolymères d'éthylène et d'anhydride maléique,
- les homopolymères d'acrylates d'alkyle et les homopolymères de méthacrylates d'alkyle tels que le produit proposé sous la dénomination MICROPEARL® RQ 750 par la Société MATSUMOTO ou le produit proposé sous la dénomination LUHYDRAN® A 848 S par la Société BASF,
- les copolymères d'esters acryliques tels que par exemple les copolymères d'acrylates d'alkyle et de méthacrylates d'alkyle tels que les produits proposés par la Société ROHM & HAAS sous les dénominations PRIMAL AC-261 K et EUDRAGIT NE 30 D, par la Société BASF sous les dénominations ACRONAL® 601, LUHYDRAN® LR 8833 ou 8845, et par la Société HOECHST sous les dénominations APPRETAN® N 9213 ou N 9212,
- les copolymères d'acrylonitrile et d'un monomère non ionique choisi par exemple parmi le butadiène et les (méth) acrylates d'alkyle ; on peut citer les produits proposés sous les dénominations NIPOL® LX 531 B par la Société NIPPON ZEON ou ceux proposés sous la dénomination CJ 0610 B par la Société ROHM & HAAS,
- les polyuréthannes tels que les produits proposés sous les dénominations ACRYSOL® RM 1020 OU ACRYSOL® RM 2020 par la Société ROHM & HAAS, les produits URAFLEX® XP 401 UZ, URAFLEX® XP et 402 UZ par la Société DSM RESINS,
- les copolymères d'acrylate d'alkyle et d'uréthanne tels que le produit 8538-33 commercialisé par la Société NATIONAL STARCH,
- les polyamides tels que le produit ESTAPOR® LO 11 proposé par la Société RHONE POULENC,
- les gommes de guar non ioniques chimiquement modifiées ou non modifiées.

Les gommes de guar non ioniques non modifiées sont par exemple les produits vendus sous la dénomination VIDOGUM® GH 175 par la Société UNIPECTINE et sous la dénomination JAGUAR® C par la Société MEYHALL. Les gommes de guar non ioniques modifiées utilisables selon l'invention sont de préférence modifiées par des groupements hydroxyalkyle en C₁₋₈. On peut mentionner à titre d'exemple les groupements hydroxyméthyle, hydroxyéthyle, hydroxypropyle et hydroxybutyle.

Ces gommes de guar sont bien connues dans la technique et peuvent par exemple être préparées par réaction des oxydes d'alcènes correspondants, tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les dénominations commerciales JAGUAR® HP8, JAGUAR® HP60 et JAGUAR® HP120, JAGUAR® DC 293 et JAGUAR® HP 105 par la Société MEYHALL, ou sous la dénomination GALACTASOL® 4H4FD2 par la Société AQUALON.

Selon l'invention, on peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les demandes de brevets US 4 693 935, US 4 728 571 et US 4 972 037.

Ces polymères sont de préférence anioniques ou non ioniques.

De tels polymères sont par exemple les copolymères susceptibles d'être obtenus par polymérisation radicalaire à partir du mélange de monomère formé,
a) de 50 à 90% en poids d'acrylate de tertiobutyle,
b) de 0 à 40% en poids d'acide acrylique,
c) de 5 à 40% en poids d'un macromère siliconé de formule : 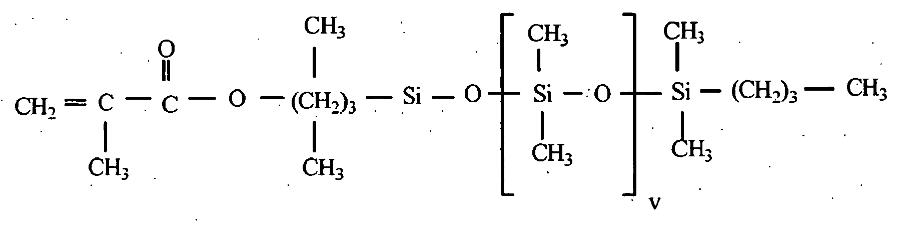
avec v étant un nombre allant de 5 à 700, les pourcentages en poids calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser comme polymères fixants des polyuréthanes fonctionnalisés ou non, siliconés ou non.

Les polyuréthanes particulièrement visés par la présente invention sont ceux décrits dans les demandes de brevets EP 0 751 162, EP 0 637 600, FR 2 743 297 et EP 0 648 485 de la demanderesse, ainsi que la demande de brevet EP 0 656 021 ou WO 94/03510 de la Société BASF et EP 0 619 111 de la Société NATIONAL STARCH.

A titre de polyuréthane fixant non siliconé on peut citer le polymère LUVISET PUR proposé par la société BASF. A titre de polyuréthane fixant siliconé on peut citer le polymère LUVISET Si PUR proposé également par la société BASF.

Le polymère fixant est de préférence choisi parmi les polyuréthanes siliconés ou non, les polyesters sulfoniques linéaires, les copolymères acryliques à blocs ramifiés, et les copolymères octylacrylamide/acrylates/butylaminoéthylméthacrylates.

Les polymères fixants particulièrement préférés peuvent ainsi être choisis parmi l'AMPHOMER de NATIONAL STARCH, le LUVISET Si Pur de BASF, le FIXATE G100 de NOVEON, le MEXOMERE PW de CHIMEX, l'AQ 55S d'EASTMAN.

De préférence, le polymère fixant représente de 0,1 à 20% en poids, de préférence de 1 à 12% en poids par rapport au poids total de la composition cosmétique.

Les compositions peuvent contenir un ou plusieurs agents protecteurs des fibres capillaires.

Les agents protecteurs des fibres capillaires peuvent être tout agent actif utile pour prévenir ou limiter les dégradations dues aux agressions physiques ou chimiques.

Ainsi, l'agent protecteur des fibres capillaires peut être choisi parmi les filtres UV organiques hydrosolubles, liposolubles, les agents antiradicalaires, les agents antioxydants, les vitamines, les provitamines, les cires végétales, les céramides, les protéines ainsi que leurs mélanges.

Les filtres UV organiques (systèmes filtrant les radiations UV) sont notamment choisis parmi les filtres hydrosolubles ou liposolubles, siliconés ou non siliconés et les nanoparticules d'oxydes minéraux dont la surface a éventuellement été traitée pour la rendre hydrophile ou hydrophobe.

Les filtres UV organiques hydrosolubles peuvent être choisis parmi par exemple, l'acide para-aminobenzoïque et ses sels, l'acide anthranilique et ses sels, l'acide salicylique et ses sels, l'acide p-hydroxycinnamique et ses sels, les dérivés sulfoniques de benz-x-azole (benzothioazoles, benzimidazoles, benzoxazoles) et leurs sels, les dérivés sulfoniques de la benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole.

On peut également utiliser des polymères hydrophiles présentant, en outre et de par leur nature chimique, des propriétés de photoprotection contre le rayonnement UV. On peut citer les polymères comportant des groupements benzylidène camphre et/ou benzotriazole, substitués par des groupements sulfoniques ou ammonium quaternaires.

Comme filtres UV organiques liposolubles (ou lipophiles) convenant à une mise en oeuvre dans la présente invention, on peut notamment citer : les dérivés d'acide p-aminobenzoïque, tels que les esters ou amides d'acide p-aminobenzoïque ; les dérivés d'acide salicylique tels que les esters; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'ester éthylique d'acide urocanique ; les benzotriazoles ; les dérivés d'hydroxyphényltriazine ; les bis-résorcinol-dialkylaminotriazine ; et leurs mélanges.

Le filtre UV liposoluble (ou lipophile) selon l'invention est de préférence choisi parmi : l'octyl salicylate ; le 4-tertiobutyl 4'-méthoxydibenzoylméthane (PARSOL 1789 de GIVAUDAN); l'octocrylène ; le 4-méthoxy cinnamate de 2-éthylhexyl (PARSOL MCX) et le composé de formule (XIII) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, décrit dans la demande de brevet EP-A-0 392 883 : 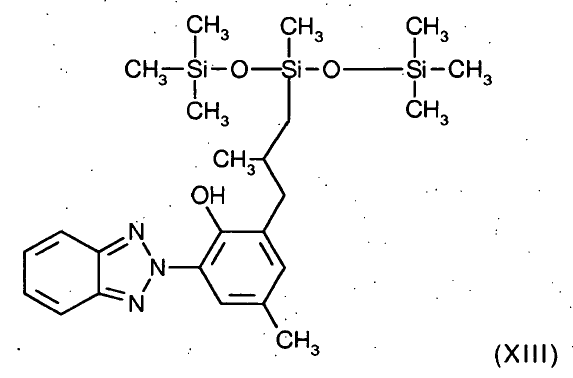 D'autres filtres UV particulièrement préférés selon l'invention sont les dérivés de benzophénones tels que l'UVINUL MS 40 (Acide 2-hydroxy 4-méthoxybenzophénone-5-sulfonique) et l'UVINUL M40 (2-hydroxy-4-méthoxybenzophénone) commercialisés par BASF, les dérivés de benzalmalonates tels que le PARSOL SLX (poly diméthyl/méthyl (3(4-(2,2-bis-éthoxycarbonylvinyl)-phénoxy)-propényl) siloxane) commercialisé par GIVAUDAN-ROURE, les dérivés de benzylidènecamphre tels que le MEXORYL SX (acide b-b'camphosulfonique [1-4 divinylbenzène]) fabriqué par la société CHIMEX, les dérivés de benzimidazole tels que l'EUSOLEX 232 (acide 2-phényl-benzimidazol-5-sulfonique) commercialisé par MERCK.

Le pH peut être ajusté à la valeur désirée au moyen d'agents acidifiants ou alcalinisants bien connus de l'état de la technique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante : 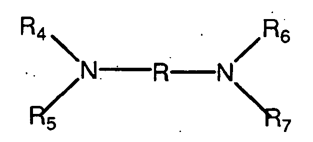 dans laquelle R est un reste propylène éventuellement substitué par un groupement hydroxyle ou un radical alkyle en C1-C4 ; R4, R5, R6 et R7, identiques ou différents, représentent un atome d'hydrogène, un radical alkyle en C1-C4 ou hydroxyalkyle en C1-C4. De préférence, une triéthanolamine est utilisée comme agent alcalinisant dans les compositions selon l'invention.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique ou des acides sulfoniques.

On peut également incorporer dans les compositions cosmétiques de l'invention des colorants directs.

Comme colorants directs utilisables selon la présente invention, on peut citer les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

On peut également incorporer dans les compositions cosmétiques de l'invention des conservateurs et/ou des agents anti-corrosion.

Parmi les conservateurs on peut citer l'acide sorbique et ses sels, les esters d'acide parahydroxybenzoïque, le phénoxyéthanol.

Parmi les agents anticorrosion utilisables selon l'invention, on peut citer les cyclohexylamines, le phosphate de diammonium, l'oxalate de dilithium, le diméthylamino méthylpropanol, l'oxalate de dipotassium le phosphate de dipotassium, un phosphate disodique, un pyrophosphate disodique, un tetrapropényl succinate disodique, un phosphate de hexoxyéthyl diéthylammonium, le nitrométhane, un silicate de potassium, un aluminate de sodium, un hexamétaphosphate de sodium, un métasilicate de sodium, un molybdate de sodium, un nitrite de sodium, un oxalate de sodium, un silicate de sodium, une stéaramidopropyl diméthicone, un pyrophosphate de tétrapotassium, et la triisopropanolamine,

Ainsi, de préférence, la composition cosmétique décrite ci-dessus comprend en outre un composé choisi parmi les polymères fixants, les agents alcalinisant, les agents acidifiants, les solvants organiques, les parfums, les agents conservateurs, les agents absorbants les UV, et les agents colorants, les agents anti-corrosion, et leurs mélanges.

La composition cosmétique selon l'invention peut comprendre de l'eau.

Le milieu cosmétiquement acceptable de la composition selon l'invention peut comprendre au moins un solvant organique.

Au sens de la présente invention on entend par solvant organique un composé organique liquide à la température de 25°C et à la pression atmosphérique (760 mm de mercure (Hg))

Le solvant organique de la composition cosmétique est généralement choisi parmi les alcools C₁-C₆ de préférence les alcanols tels que l'éthanol, le propanol et l'isopropanol, les polyols tels que le glycérol, le propylène glycol et le pentanediol, l'alcool benzylique, et leurs mélanges.

De préférence, le solvant organique est l'éthanol.

En général, lorsqu'il est présent, le solvant organique représente de 1 à 98% et de préférence, de 20 à 95% en poids par rapport au poids total de la composition.

Le dispositif de pulvérisation d'une composition selon l'invention peut notamment être utilisée en application non rincée aussi bien sur des cheveux secs que sur des cheveux humides.

Les exemples suivants illustrent la présente invention.

### Exemples de formulation

### Exemple 1

### Composition 1

| | |
|---|---|
| Polyphénylméthylsiloxane commercialisé par DOW CORNING sous la dénomination DOW CORNING 556 COSMETIC GRAD FLUID | 10,00g |
| Ethylhexyl methoxycinnamate commercialisé par ROCHE VITAMINS sous la dénomination PARSOL MCX | 0,05g |
| Parfum | 0,10g |
| Alcool éthylique absolu qsp | 100g |

La composition 1 est conditionnée dans un dispositif de pulvérisation tel que défini ci-dessus. Ce dispositif comprend une membrane comprenant elle-même des perforations qui présentent une surface en coupe transversale en forme de disque circulaire dont le diamètre varie de 4 à 150µm sur la surface externe de la membrane, faisant face à l'environnement externe, et sur la surface interne, faisant face à l'intérieur du récipient, une surface en coupe transversale en forme de disque circulaire dont le diamètre varie de 2 à 50µm. Ce dispositif comprend un actionneur piézo-électrique capable de faire vibrer la membrane ainsi qu'une pile électrique destinée à fournir l'énergie nécessaire à l'actionneur piézo-électrique. Les perforations du dispositif ont une conicité inversée.

Lors de l'utilisation du dispositif, la composition cosmétique émerge sous forme de gouttelettes dont le diamètre moyen est compris entre 20 et 100µm.

### Exemple 2

### Composition 2

| | |
|---|---|
| Myristate d'isopropyle | 4,00g |
| Huile de vaseline | 4,00g |
| Ethylhexyl methoxycinnamate commercialisé par ROCHE VITAMINS sous la dénomination PARSOL MCX | 0,05g |
| Parfum | 0,10g |
| Alcool éthylique absolu qsp | 100g |

La composition 2 est conditionnée dans un dispositif de pulvérisation identique à celui défini ci-dessus dans l'exemple 1.

Le dispositif de pulvérisation décrit dans l'exemple 1, comprenant la composition 1, ou 2, telle que définie ci-dessus permet d'obtenir une intensité et/ou une durabilité de la brillance de la coiffure qui ne peut être atteinte habituellement avec les dispositifs connus sans propulseurs tels que les flacons pompes.

L'application peut se faire aussi bien sur cheveux humides que secs.

## Revendications

1. Dispositif de pulvérisation d'une composition cosmétique
comprenant un récipient équipé d'un mécanisme de pulvérisation
comprenant :
- une membrane perforée, les perforations de la membrane faisant
communiquer l'intérieur du récipient avec l'environnement externe,
- un actionneur pour faire vibrer la membrane, **caractérisé par** la présence dans le recipient d'une composition cosmétique liquide, comprenant, dans un milieu cosmétiquement acceptable, 0,1 à 40 % en poids d'au moins une silicone, par rapport au poids total de la composition cosmétique, et/ou 0,1 à 40 % en poids d'au moins un corps gras non siliconé, par rapport au poids total de la composition cosmétique, ladite composition cosmétique étant contenue dans ledit récipient,
d'un moyen pour amener ladite composition cosmétique liquide au contact d'une surface interne de la membrane, ladite composition cosmétique, sous l'effet des vibrations de la membrane s'écoulant à travers les perforations jusqu'à une surface externe de la membrane, d'où elle émerge sous forme de gouttelettes.

2. Dispositif de pulvérisation d'une composition selon la
revendication 1, **caractérisé en ce que** les perforations dans la
membrane ont une conicité inversée, c'est-à-dire une surface en coupe
transversale plus grande sur la surface externe de la membrane, faisant
face à l'environnement externe, que sur la surface interne, faisant face à
l'intérieur du récipient.

3. Dispositif de pulvérisation d'une composition selon la
revendication 1 ou 2, **caractérisé en ce qu'**il comprend en outre un
moyen de décalage de pression fournissant une pression réduite au
liquide en contact avec la surface interne de la membrane.

4. Dispositif de pulvérisation d'une composition selon la
revendication 3, **caractérisé en ce que** la pression réduite varie de la
pression ambiante jusqu'à la pression à laquelle l'air est aspiré à travers les perforations de la membrane en contact avec la composition.

5. Dispositif de pulvérisation d'une composition selon la revendication 1 ou 2, **caractérisé en ce que** les perforations, sur la surface externe de la membrane, ne se touchent pas.

6. Dispositif de pulvérisation d'une composition selon l'une quelconque des revendications précédentes, **caractérisé en ce que** l'actionneur est un actionneur piézo-électrique.

7. Dispositif de pulvérisation d'une composition selon l'une quelconque des revendications précédentes, **caractérisé en ce que** le moyen pour amener la composition cosmétique liquide à la surface de la membrane comprend un mécanisme d'alimentation par capillarité.

8. Dispositif de pulvérisation d'une composition selon l'une quelconque des revendications précédentes, **caractérisé en ce que** le moyen pour amener la composition cosmétique liquide à la surface de la membrane comprend un mécanisme d'alimentation à générateur de bulles.

9. Dispositif de pulvérisation d'une composition selon l'une quelconque des revendications précédentes, **caractérisé en ce que** toutes les perforations ont une conicité inversée.

10. Dispositif de pulvérisation d'une composition selon l'une quelconque des revendications 1 à 8, **caractérisé en ce que** la membrane comporte de plus des perforations de conicité normale.

11. Dispositif de pulvérisation d'une composition selon la revendication 10, **caractérisé en ce que** les perforations de conicité normale sont disposées autour et à l'extérieur des perforations de conicité inversée.

12. Dispositif de pulvérisation d'une composition selon l'une quelconque des revendications précédentes, **caractérisé en ce que** l'actionneur est conçu pour faire vibrer ladite membrane dans une gamme de fréquence allant de 20 KHz à 7 MHz.

13. Dispositif de pulvérisation d'une composition selon l'une quelconque des revendications précédentes, **caractérisé en ce que** la silicone est choisie parmi les silicones volatiles, linéaires ou cycliques et les silicones arylées.

14. Dispositif de pulvérisation d'une composition selon la revendication 13, **caractérisé en ce que** la silicone est une phényl triméthylsiloxy trisiloxane.

15. Dispositif de pulvérisation d'une composition selon l'une quelconque des revendications précédentes **caractérisé en ce que** la ou les silicones représentent de préférence 1 à 20% en poids par rapport au poids total de la composition.

16. Dispositif de pulvérisation d'une composition selon l'une quelconque des revendications précédentes **caractérisé en ce que** le corps gras non siliconé est choisi parmi un alcool gras, un ester gras, une huile minérale, végétale, animale ou synthétique ou une cire.

17. Dispositif de pulvérisation d'une composition selon la revendication 16, **caractérisé en ce que** l'ester est choisi parmi les palmitates d'éthyle, d'isopropyle, de myristyle, de cétyle, de stéaryle, le palmitate d'éthyl-2-héxyle, le palmitate de 2-octyldécyle, les myristates d'alkyles, le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécylé et l'isononanate d'isononyle, l'octanoate de cétyle.

18. Dispositif de pulvérisation d'une composition selon l'une quelconque des revendications précédentes, **caractérisé en ce que** le corps gras non siliconé est l'huile de vaseline.

19. Dispositif de pulvérisation d'une composition selon l'une quelconque des revendications précédentes, **caractérisé en ce que** le ou les corps gras non siliconés représentent de préférence 1 à 20% en poids par rapport au poids total de la composition cosmétique.

20. Dispositif de pulvérisation d'une composition selon l'une quelconque des revendications précédentes, **caractérisé en ce que** la composition cosmétique comprend de l'eau.

21. Dispositif de pulvérisation d'une composition selon l'une quelconque des revendications précédentes, **caractérisé en ce qu'**elle comprend un ou plusieurs additifs choisis parmi les polymères fixants, les agents alcalinisants, les agents acidifiants, les parfums, les agents conservateurs, les agents absorbants les UV, les agents colorants, les agents anticorrosion et leurs mélanges.

22. Dispositif de pulvérisation d'une composition selon l'une quelconque des revendications précédentes, **caractérisé en ce que** le milieu cosmétiquement acceptable comprend au moins un solvant organique choisi parmi les alcools en C₁-C₆ de préférence les alcanols tels que l'éthanol, le propanol et l'isopropanol, les polyols tels que le glycérol, le propylène glycol et le pentanediol, l'alcool benzylique, et leurs mélanges.

## Patentansprüche

1. Vorrichtung zum Versprühen einer kosmetischen Zusammensetzung, umfassend einen Behälter, der mit einem Sprühmechanismus ausgestattet ist, umfassend:
- eine perforierte Membran, wobei die Perforationen der Membran das Innere des Behälters mit der äußeren Umgebung in Verbindung bringen,
- einen Aktor, um die Membran schwingen zu lassen, **gekennzeichnet durch** die Gegenwart einer flüssigen kosmetischen Zusammensetzung, umfassend, in einem kosmetisch verträglichen Medium, 0,1 bis 40 Gew.-% mindestens eines Silikons, bezogen auf das Gesamtgewicht der kosmetischen Zusammensetzung, und/oder 0,1 bis 40 Gew.-% mindestens einer silikonfreien Fettsubstanz, bezogen auf das Gesamtgewicht der kosmetischen Zusammensetzung, wobei die kosmetische Zusammensetzung in dem Behälter enthalten ist,
eines Mittels zum Inkontaktbringen der flüssigen kosmetischen Zusammensetzung mit einer inneren Oberfläche der Membran,
wobei die kosmetische Zusammensetzung unter Einwirkung der Schwingungen der Membran **durch** die Perforationen bis zu einer äußeren Oberfläche der Membran fließt, wo sie in Form von Tröpfchen austritt.

2. Vorrichtung zum Versprühen einer Zusammensetzung nach Anspruch 1, **dadurch gekennzeichnet, dass** die Perforationen in der Membran eine umgekehrte Konizität aufweisen, das heißt, eine Querschnittsoberfläche, die an der äußeren Oberfläche der Membran, die der äußeren Umgebung zugewandt ist, größer ist als an der inneren Oberfläche, die dem Inneren des Behälters zugewandt ist.

3. Vorrichtung zum Versprühen einer Zusammensetzung nach Anspruch 1 oder 2, **dadurch gekennzeichnet, dass** sie ferner ein Mittel zur Herstellung einer Druckdifferenz umfasst, das für die Flüssigkeit in Kontakt mit der inneren Oberfläche der Membran einen reduzierten Druck bereitstellt.

4. Vorrichtung zum Versprühen einer Zusammensetzung nach Anspruch 3, **dadurch gekennzeichnet, dass** der reduzierte Druck vom Umgebungsdruck bis zu dem Druck variiert, bei dem die Luft durch die Perforationen der Membran in Kontakt mit der Zusammensetzung gesaugt wird.

5. Vorrichtung zum Versprühen einer Zusammensetzung nach Anspruch 1 oder 2, **dadurch gekennzeichnet, dass** sich die Perforationen auf der äußeren Oberfläche der Membran nicht berühren.

6. Vorrichtung zum Versprühen einer Zusammensetzung nach einem der vorhergehenden Ansprüche, **dadurch gekennzeichnet, dass** der Aktor ein Piezoaktor ist.

7. Vorrichtung zum Versprühen einer Zusammensetzung nach einem der vorhergehenden Ansprüche, **dadurch gekennzeichnet, dass** das Mittel zur Beförderung der flüssigen kosmetischen Zusammensetzung an die Oberfläche der Membran einen Zufuhrmechanismus durch Kapillarwirkung umfasst.

8. Vorrichtung zum Versprühen einer Zusammensetzung nach einem der vorhergehenden Ansprüche, **dadurch gekennzeichnet, dass** das Mittel zur Beförderung der flüssigen kosmetischen Zusammensetzung an die Oberfläche der Membran einen Zufuhrmechanismus durch einen Blasengenerator umfasst.

9. Vorrichtung zum Versprühen einer Zusammensetzung nach einem der vorhergehenden Ansprüche, **dadurch gekennzeichnet, dass** alle Perforationen eine umgekehrte Konizität haben.

10. Vorrichtung zum Versprühen einer Zusammensetzung nach einem der Ansprüche 1 bis 8, **dadurch gekennzeichnet, dass** die Membran außerdem Perforationen mit normaler Konizität umfasst.

11. Vorrichtung zum Versprühen einer Zusammensetzung nach Anspruch 10, **dadurch gekennzeichnet, dass** die Perforationen mit normaler Konizität um die Perforationen mit umgekehrter Konizität herum und außerhalb dieser angeordnet sind.

12. Vorrichtung zum Versprühen einer Zusammensetzung nach einem der vorhergehenden Ansprüche, **dadurch gekennzeichnet, dass** der Aktor konzipiert ist, um die Membran in einem Frequenzbereich von 20 KHz bis 7 MHz schwingen zu lassen.

13. Vorrichtung zum Versprühen einer Zusammensetzung nach einem der vorhergehenden Ansprüche, **dadurch gekennzeichnet, dass** das Silikon ausgewählt ist aus linearen oder zyklischen flüchtigen Silikonen und arylierten Silikonen.

14. Vorrichtung zum Versprühen einer Zusammensetzung nach Anspruch 13, **dadurch gekennzeichnet, dass** das Silikon ein Phenyltrimethylsiloxytrisiloxan ist.

15. Vorrichtung zum Versprühen einer Zusammensetzung nach einem der vorherigen Ansprüche, **dadurch gekennzeichnet, dass** das Silikon oder die Silikone bevorzugt 1 bis 20 Gew.-%, bezogen auf das Gesamtgewicht der Zusammensetzung, ausmachen.

16. Vorrichtung zum Versprühen einer Zusammensetzung nach einem der vorhergehenden Ansprüche, **dadurch gekennzeichnet, dass** die silikonfreie Fettsubstanz ausgewählt ist aus einem Fettalkohol, einem Fettester, einem mineralischen, pflanzlichen, tierischen oder synthetischen Öl oder einem Wachs.

17. Vorrichtung zum Versprühen einer Zusammensetzung nach Anspruch 16, **dadurch gekennzeichnet, dass** der Ester ausgewählt ist aus Ethyl-, Isopropyl-, Myristyl-, Cetyl-, Stearylpalmitaten, Ethyl-2-hexylpalmitat, 2-Octyldecylpalmitat, Alkylmyristaten, Isopropyl-, Butyl-, Cetyl-, 2-Octyldodecylmyristat, Hexylstearat, Butylstearat, Isobutylstearat; Dioctylmalat, Hexyllaurat, 2-Hexyldecyllaurat und Isononylisononanat, Cetyloctanoat.

18. Vorrichtung zum Versprühen einer Zusammensetzung nach einem der vorhergehenden Ansprüche, **dadurch gekennzeichnet, dass** die silikonfreie Fettsubstanz Vaselinöl ist.

19. Vorrichtung zum Versprühen einer Zusammensetzung nach einem der vorhergehenden Ansprüche, **dadurch gekennzeichnet, dass** die silikonfreie(n) Fettsubstanz(en) 1 bis 20 Gew.-%, bezogen auf das Gesamtgewicht der kosmetischen Zusammensetzung, ausmachen.

20. Vorrichtung zum Versprühen einer Zusammensetzung nach einem der vorhergehenden Ansprüche, **dadurch gekennzeichnet, dass** die kosmetische Zusammensetzung Wasser umfasst.

21. Vorrichtung zum Versprühen einer Zusammensetzung nach einem der vorhergehenden Ansprüche, **dadurch gekennzeichnet, dass** sie einen oder mehrere Zusatzstoffe umfasst, ausgewählt aus festigenden Polymeren, Alkalisierungsmitteln, Säurebildnern, Parfums, Konservierungsmitteln, UV-Absorbern, Farbmitteln, Korrosionsschutzmitteln und deren Gemischen.

22. Vorrichtung zum Versprühen einer Zusammensetzung nach einem der vorhergehenden Ansprüche, **dadurch gekennzeichnet, dass** das kosmetisch verträgliche Medium mindestens ein organisches Lösemittel umfasst, ausgewählt aus C₁-C₆-Alkoholen, bevorzugt Alkanolen, wie etwa Ethanol, Propanol und Isopropanol, Polyolen, wie etwa Glycerol, Propylenglycol und Pentandiol, Benzylalkohol und deren Gemischen.

## Claims

1. Device for spraying a cosmetic composition comprising a container equipped with a spraying mechanism comprising:
- a perforated membrane, the perforations of the membrane enabling communication of the interior of the container with the external environment,
- an actuator to make the membrane vibrate, **characterized by** the presence in the container of a liquid cosmetic composition, comprising, in a cosmetically acceptable medium, 0.1% to 40% by weight of at least one silicone, relative to the total weight of the cosmetic composition, and/or 0.1% to 40% by weight of at least one non-silicone fatty substance, relative to the total weight of the cosmetic composition, said cosmetic composition being contained in said container,
of a means for bringing said liquid cosmetic composition into contact with an inner surface of the membrane,
said cosmetic composition, under the effect of the vibrations of the membrane, flowing through the perforations up to an outer surface of the membrane, where it emerges in the form of droplets.

2. Device for spraying a composition according to Claim 1, **characterized in that** the perforations in the membrane have an inverse conicity, i.e. a cross-sectional surface that is greater on the outer surface of the membrane, facing the external environment, than on the inner surface, facing the interior of the container.

3. Device for spraying a composition according to Claim 1 or 2, **characterized in that** it also comprises a pressure shift means which provides a reduced pressure to the liquid in contact with the inner surface of the membrane.

4. Device for spraying a composition according to Claim 3, **characterized in that** the reduced pressure varies from ambient pressure up to the pressure at which the air is sucked through the perforations of the membrane in contact with the composition.

5. Device for spraying a composition according to Claim 1 or 2, **characterized in that** the perforations, on the outer surface of the membrane, do not touch each other.

6. Device for spraying a composition according to any one of the preceding claims, **characterized in that** the actuator is a piezoelectric actuator.

7. Device for spraying a composition according to any one of the preceding claims, **characterized in that** the means for bringing the liquid cosmetic composition to the surface of the membrane comprises a feed mechanism by capillary action.

8. Device for spraying a composition according to any one of the preceding claims, **characterized in that** the means for bringing the liquid cosmetic composition to the surface of the membrane comprises a feed mechanism with a bubble generator.

9. Device for spraying a composition according to any one of the preceding claims, **characterized in that** all the perforations have an inverse conicity.

10. Device for spraying a composition according to any one of Claims 1 to 8, **characterized in that** the membrane furthermore comprises perforations of normal conicity.

11. Device for spraying a composition according to Claim 10, **characterized in that** the perforations of normal conicity are arranged around and to the exterior of the perforations of inverse conicity.

12. Device for spraying a composition according to any one of the preceding claims, **characterized in that** the actuator is designed to make said membrane vibrate in a frequency range extending from 20 KHz to 7 MHz.

13. Device for spraying a composition according to any one of the preceding claims, **characterized in that** the silicone is chosen from linear or cyclic volatile silicones and aryl silicones.

14. Device for spraying a composition according to Claim 13, **characterized in that** the silicone is a phenyltrimethylsiloxytrisiloxane.

15. Device for spraying a composition according to any one of the preceding claims, **characterized in that** the silicone or silicones preferably represent 1% to 20% by weight relative to the total weight of the composition.

16. Device for spraying a composition according to any one of the preceding claims, **characterized in that** the non-silicone fatty substance is chosen from a fatty alcohol, a fatty ester, a mineral, vegetable, animal or synthetic oil or a wax.

17. Device for spraying a composition according to Claim 16, **characterized in that** the ester is chosen from ethyl, isopropyl, myristyl, cetyl and stearyl palmitates, 2-ethylhexyl palmitate, 2-octyldecyl palmitate, alkyl myristates, isopropyl, butyl, cetyl and 2-octyldodecyl myristate, hexyl stearate, butyl stearate, isobutyl stearate; dioctyl malate, hexyl laurate, 2-hexyldecyl laurate and isononyl isononanoate, and cetyl octanoate.

18. Device for spraying a composition according to any one of the preceding claims, **characterized in that** the non-silicone fatty substance is liquid petroleum jelly.

19. Device for spraying a composition according to any one of the preceding claims, **characterized in that** the non-silicone fatty substance or substances preferably represent 1% to 20% by weight relative to the total weight of the cosmetic composition.

20. Device for spraying a composition according to any one of the preceding claims, **characterized in that** the cosmetic composition comprises water.

21. Device for spraying a composition according to any one of the preceding claims, **characterized in that** it comprises one or more additives chosen from fixing polymers, basifying agents, acidifying agents, fragrances, preservatives, UV absorbents, colorants, anticorrosion agents and mixtures thereof.

22. Device for spraying a composition according to any one of the preceding claims, **characterized in that** the cosmetically acceptable medium comprises at least one organic solvent chosen from C₁-C₆ alcohols, preferably alkanols such as ethanol, propanol and isopropanol, polyols such as glycerol, propylene glycol and pentanediol, benzyl alcohol and mixtures thereof.
